# FASCICULE DE BREVET EUROPEEN

(11) **EP 2 235 192 B1**
(45) Date de publication et mention de la délivrance du brevet: **13.07.2011**
(21) Numéro de dépôt: 08872760.7
(22) Date de dépôt: 16.12.2008
(51) Int. Cl.: C12P 7/10, C12N 9/36

(54) **Complémentation du sécrétome de Trichoderma reesei limitant les contaminations microbiologiques dans le cadre de la production d'éthanol par fermentation**
Komplementierung des Sekretoms von Trichoderma reesei zur Begrenzung mikrobiologischer Verunreinigungen bei der fermentativen Herstellung von Ethanol
Complementation of the Trichoderma reesei secretome limiting microbiological contaminations in the context of the fermentative production of ethanol

(30) Priorité: 20.12.2007 FR 0709120
(43) Date de publication de la demande: 06.10.2010
(73) Titulaire: IFP Energies nouvelles, 92852 Rueil-Malmaison Cedex (FR)
(72) Inventeur: LOPES FERREIRA, Nicolas, F-28210 Croisilles (FR); MARGEOT, Antoine, F-75020 Paris (FR)
(86) Numéro de dépôt international: PCT/FR2008/001743
(87) Numéro de publication internationale: WO 2009/106703

(56) Documents cités:
- WO-A-03/078644
- WO-A-2007/109750
- BAYROCK D P ET AL: "Control of Lactobacillus contaminants in continuous fuel ethanol fermentations by constant or pulsed addition of penicillin G." APPLIED MICROBIOLOGY AND BIOTECHNOLOGY, vol. 62, no. 5-6, octobre 2003 (2003-10), pages 498-502, XP002498148 ISSN: 0175-7598
- BARON M ET AL: "EFFICIENT SECRETION OF HUMAN LYSOZYME FUSED TO THE SH BLE PHLEOMYCIN RESISTANCE PROTEIN BY THE FUNGUS TOLYPOCLADIUM-GEODES" JOURNAL OF BIOTECHNOLOGY, vol. 24, no. 3, 1992, pages 253-266, XP002498149 ISSN: 0168-1656
- SPENCER A ET AL: "Expression, Purification, and Characterization of the Recombinant Calcium-Binding Equine Lysozyme Secreted by the Filamentous FungusAspergillus niger:Comparisons with the Production of Hen and Human Lysozymes" PROTEIN EXPRESSION AND PURIFICATION, ACADEMIC PRESS, SAN DIEGO, CA, vol. 16, no. 1, 1 juin 1999 (1999-06-01), pages 171-180, XP004441696 ISSN: 1046-5928

## Description

### Domaine de l'invention

La présente invention s'inscrit dans la cadre d'un procédé dit de "seconde génération" de production d'éthanol à partir de biomasse lignocellulosique, comprenant des étapes de prétraitement d'un substrat cellulosique ou lignocellulosique, d'hydrolyse enzymatique du substrat prétraité puis de fermentation alcoolique de l'hydrolysat obtenu.

### Étude de l'art antérieur

La biomasse lignocellulosique représente une des ressources renouvelables les plus abondantes sur terre, et certainement une des moins coûteuses. Les substrats considérés sont très variés, puisqu'ils concernent à la fois les substrats ligneux (feuillus et résineux), les sous-produits de l'agriculture (paille) ou ceux des industries génératrices de déchets lignocellulosiques (industries agroalimentaires, papeteries).

La biomasse lignocellulosique est composée de trois principaux polymères : la cellulose (35 à 50%), l'hémicellulose (20 à 30%) qui est un polysaccharide essentiellement constitué de pentoses et d'hexoses et la lignine (15 à 25%) qui est un polymère de structure complexe et de haut poids moléculaire, composé d'alcools aromatiques reliés par des liaisons éther

Ces différentes molécules sont responsables des propriétés intrinsèques de la paroi végétale et s'organisent en un enchevêtrement complexe.

La cellulose et éventuellement les hémicelluloses sont les cibles de l'hydrolyse enzymatique mais ne sont pas directement accessibles aux enzymes. C'est la raison pour laquelle ces substrats doivent subir un prétraitement précédant l'étape d'hydrolyse enzymatique. Le prétraitement vise à modifier les propriétés physiques et physicochimiques du matériau lignocellulosique, en vue d'améliorer l'accessibilité de la cellulose engluée dans la matrice de lignine et d'hémicellulose. Il peut également libérer les sucres contenus dans les hémicelluloses sous forme de monomères, essentiellement des pentoses, comme le xylose et l'arabinose, et des hexoses, comme le galactose, le mannose et le glucose.

Le prétraitement doit dans l'idéal être rapide et efficace, à fortes concentrations en substrats, et les pertes de matières doivent être minimales. De nombreuses technologies existent : on peut citer les cuissons acides, les cuissons alcalines, l'explosion à la vapeur (Pourquié, J. et Vandecasteele, J.P. (1993) Conversion de la biomasse lignocellulosique par hydrolyse enzymatique et fermentation. Biotechnologie, 4e édition, René Scriban, coordinateur Lavoisier TEC & DOC, Paris, 677-700.), les procédés Organosolv, ou encore les technologies bi-vis combinant des actions thermique, mécanique et chimique (Ogier J.-C. et al. (1999) Production d'éthanol à partir de biomasse lignocellulosique Oil & Gas Science and Technology 54:67-94). L'efficacité du prétraitement se mesure par la susceptibilité à l'hydrolyse du résidu cellulosique et le taux de récupération des hémicelluloses. D'un point de vue économique, il semble préférable que le prétraitement conduise à une hydrolyse totale des hémicelluloses, de façon à récupérer les pentoses et éventuellement les valoriser séparément de la fraction cellulosique. Les prétraitements acides en conditions douces et par explosion à la vapeur sont bien adaptés. Ils permettent une récupération importante des sucres issus des hémicelluloses et une bonne accessibilité de la cellulose à l'hydrolyse.

Le résidu cellulosique obtenu est hydrolysé par voie enzymatique par l'utilisation d'enzymes cellulolytiques et/ou hémicellulolytiques. Des microorganismes, comme les champignons appartenant aux genres *Trichoderma, Aspergillus, Penicillium ou Schizophyllum,* ou les bactéries anaérobies appartenant par exemple au genre *Clostridium,* produisent ces enzymes, contenant notamment les cellulases et les hémicellulases, adaptées à l'hydrolyse totale de la cellulose et des hémicelluloses.

L'hydrolyse enzymatique s'effectue dans des conditions douces (température de l'ordre de 45-50°C et pH de 4,8) et est efficace. En revanche en termes de procédé, le coût des enzymes reste très élevé. De ce fait, beaucoup de travaux ont été conduits pour réduire ce coût : i) augmentation de la production d'enzymes d'abord, en sélectionnant des souches hyperproductrices et en améliorant les procédés de fermentation, ii) diminution de la quantité d'enzymes en hydrolyse ensuite, en optimisant la phase de prétraitement ou en améliorant l'activité spécifique de ces enzymes. Au cours de la dernière décennie, les principaux travaux se sont attachés à comprendre les mécanismes d'action des cellulases et d'expression des enzymes afin de faire excréter le complexe enzymatique le plus approprié à l'hydrolyse des substrats ligno-cellulosiques en modifiant les souches avec les outils de biologie moléculaire.

Les champignons filamenteux, en tant qu'organismes cellulolytiques, présentent un grand intérêt pour les industriels puisqu'ils ont la capacité de produire des enzymes extracellulaires en très grande quantité. Le microorganisme le plus utilisé pour la production de cellulases est le champignon *Trichoderma reesei.* Les souches sauvages ont la faculté de produire, en présence d'un substrat inducteur, la cellulose par exemple, un sécrétome (ensemble des protéines sécrétées) adapté à l'hydrolyse de la cellulose. Les enzymes du complexe enzymatique contiennent trois grands types d'activités : les endoglucanases, les exoglucanases et les β-glucosidases. D'autres protéines possédant des propriétés indispensables à l'hydrolyse des matériaux ligno-cellulosiques sont également produites par *Trichoderma reesei,* les xylanases par exemple. La présence d'un substrat inducteur est indispensable à l'expression des enzymes cellulolytiques et/ou hémicellulolytiques. La nature du substrat carboné a une forte influence sur la composition du complexe enzymatique. C'est le cas du xylose, qui, associé à un substrat carboné inducteur comme la cellulose ou le lactose, permet d'améliorer significativement l'activité dite xylanase.

Les techniques de génétique classique par mutagénèse ont permis la sélection de souches de *Trichoderma reesei* hyperproductrices de cellulases telles que les souches MCG77 (Gallo - brevet US 4275 167), MCG 80 (Allen, A.L. et Andreotti, R.E., Biotechnol-Bioengi 1982, 12, 451-459 1982), RUT C30 (Montenecourt, B.S. et Eveleigh, D.E., Appl. Environ. Microbiol. 1977, 34, 777-782) et CL847 (Durand et al, 1984, Proc. Colloque SFM "Génétique des microorganismes industriels". Paris. H. HESLOT Ed, pp 39-50). Les améliorations ont permis d'obtenir des souches hyperproductrices, moins sensibles à la répression catabolique sur sucres monomères notamment, glucose par exemple, que les souches sauvages.

La banalisation des techniques génétiques, visant à exprimer des gènes hétérologues au sein de ces souches fongiques, a aussi ouvert la voie vers l'utilisation de ces micro-organismes en tant qu'hôtes pour la production industrielle. Les nouvelles techniques d'études des profils enzymatiques, ont rendu possible la création de souches fongiques hôtes très efficaces pour la production d'enzymes recombinantes à l'échelle industrielle [Nevalainen, H. and Teo, V.J.S. (2003) Enzyme production in industrial fungi-molecular genetic strategies for integrated strain improvement. In Applied Mycology and Biotechnology (Vol. 3) Fungal Genomics (Arora, D.K. and Kchachatourians, G.G., eds), pp. 241-259, Elsevier Science]. Un des exemples de ce type de modifications est la production de cellulases issues de d'une souche de *T. reesei* [Harkki, A. et al. (1991) Genetic engineering of Trichoderma to produce strains with novel cellulase profiles. Enzyme Microb. Technol. 13, 227-233 ; Karhunen, T. et al. (1993) High frequency one-step gene replacement in Trichoderma reesei. I. Endoglucanase I overproduction. Mol. Gen. Genet. 241, 515-522].

Les sucres obtenus par hydrolyse de la biomasse lignocellulosique sont des pentoses (xylose et arabinose principalement), des disaccharides (cellobiose) et du glucose. Ce dernier est facilement transformé en éthanol par la levure *S. cerevisiae* utilisée par l'ensemble des industries de la fermentation alcoolique. Actuellement, aucun autre micro-organisme n'atteint ses performances sur glucose en conditions non stériles, à savoir un rendement de l'ordre de 0,47 g d'éthanol par gramme de glucose, une productivité supérieure ou égale à 5 g/l×h, et des concentrations finales en éthanol voisines de 10% en volume. *S. cerevisiae* présente de nombreux atouts supplémentaires résultant de nombreuses années de sélection : résistance à l'éthanol, mise en oeuvre industrielle aisée, etc. Par contre, les pentoses sont rarement fermentés par les micro-organismes et, lorsqu'ils le sont, les performances sont médiocres. Ces dernières années, beaucoup de travaux ont porté sur la recherche et/ou l'amélioration des souches fermentant activement les pentoses en éthanol. Quatre types de micro-organismes ont été étudiés : les levures fermentant naturellement les pentoses, des souches recombinées de *S. cerevisiae,* des bactéries thermophiles ou mésophiles utilisant les pentoses.

La fermentation alcoolique en conditions non stériles présente un risque important de contaminations du fermenteur par des micro-organismes opportunistes. Les sources de contamination peuvent être de nature vivante ou non-vivante. Toutefois, nous ne nous attacherons ici qu'aux sources de contamination vivante. Parmi ces sources, on retrouve principalement les levures et les bactéries. Ces micro-organismes utilisent les nutriments présents, dont la source de carbone, et sont responsables de la formation de co-produits indésirables comme l'acide lactique, l'acide acétique ou même de l'acétone et du butanol. On retrouve ce genre de micro-organismes partout où les conditions permettent leur croissance c'est-à-dire en présence d'un minimum de nutriments. Mentionnons ici que leurs exigences nutritionnelles sont : une source de carbone (généralement les sucres), une source d'acides aminés (constituants des protéines), certaines vitamines et oligo-éléments.

De plus, au sein des matières premières énergétiques envisagées, la paille de blé par exemple, il est tout a fait probable de retrouver des micro-organismes capables de contaminer le procédé.

Ainsi, dans le procédé actuel et non stérile de production d'éthanol de seconde génération, deux étapes sont sensibles à une éventuelle contamination microbiologique : l'étape d'hydrolyse enzymatique et l'étape de fermentation. Les solutions connues actuellement pour lutter contre la contamination lactique consistent à abaisser le pH à une valeur favorisant le développement des levures au détriment des bactéries lactiques. Les levures sont cependant moins actives à ces pH acides. Une autre possibilité est d'introduire des inhibiteurs de contamination bactérienne, comme par exemple du fluor, des antibiotiques ou des sulfites, lors de l'étape de fermentation alcoolique. C'est en effet, lors de cette étape du procédé que la concentration en contaminants est la plus importante. L'utilisation d'anti-bactériens classiques a un coût relativement élevé et nécessite d'effectuer des relances assez fréquentes du processus de fermentation.

Limiter les risques de contamination constitue un gain potentiel de temps et d'argent pour des procédés industriels d'une telle ampleur et aucune solution n'est à négliger pour remédier à ce problème.

La contamination bactérienne est en effet un problème majeur dans la production d'éthanol par fermentation. Les bactéries sont présentes naturellement au sein de l'outil de production et utilisent les nutriments présents dans le milieu, entrant par conséquence en concurrence avec les levures utilisées dans le procédé. La croissance et la viabilité des cellules de levures sont donc fortement affectées par la présence de ces bactéries et le rendement final en alcool en est également réduit.

En général, les bactéries lactiques fermentent des sucres présents dans les moûts de fermentation et leur croissance est favorisée par des conditions anaérobies. Elles se développent généralement à un pH de 5,5 mais peuvent survivre à un pH aussi bas que pH 3,0. Ces bactéries opportunistes peuvent se développer sur une large gamme de température et sont tolérantes à de fortes concentrations en alcool dans le milieu. La présence des bactéries dans les procédés de production d'éthanol de seconde génération est à proscrire. Toute amélioration du procédé conduisant à une limitation maximale de cette contamination est à prendre en considération.

### Description des figures

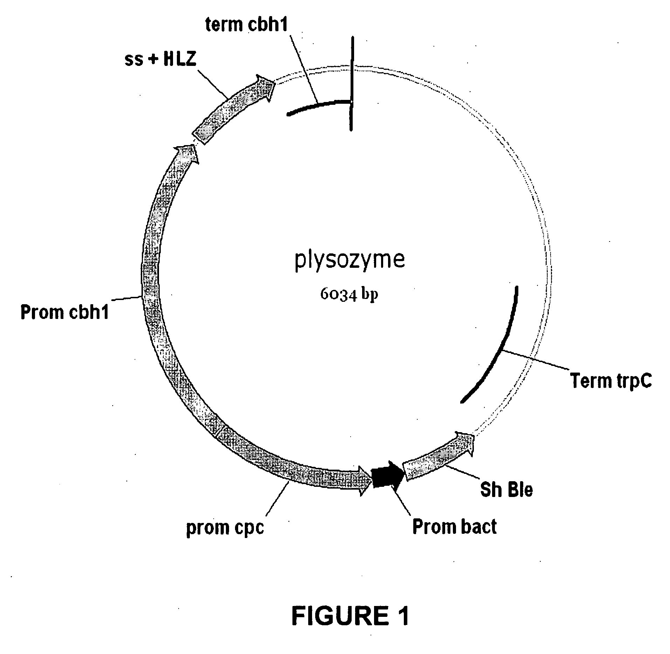

La Figure 1 donne une représentation de la carte schématique d'un plasmide pouvant être utilisé pour l'expression hétérologue du gène *h*/*z* par *T. reesei* en conditions d'induction de cellulases.

### Résumé de l'invention

La présente invention décrit un procédé de production d'éthanol à partir de biomasse lignocellulosique dans lequel on complémente le génome de *Trichoderma reesei* par le lysozyme.

### Description détaillée de l'invention

Dans le procédé de production d'éthanol de seconde génération décrit dans la présente invention, afin de prévenir les contaminations microbiologiques, on utilise pour produire les enzymes cellulolytiques et/ou hémicellulolytiques une souche fongique modifiée génétiquement appartenant au genre *Trichoderma* et surexprimant, à l'aide de techniques génétiques bien connues de l'homme de l'art, le lysozyme.

La souche fongique (microorganisme cellulolytique) appartient préférentiellement à l'espèce *Trichoderma reesei.*

L'utilisation du lysozyme, qui est une protéine ayant des propriétés anti-microbiennes, permet d'assurer une protection contre un large éventail de bactéries Gram-positives, notamment contre les bactéries lactiques. Ces bactéries se développent dans les procédés de production d'éthanol de seconde génération dès l'étape d'hydrolyse enzymatique et principalement lors de la fermentation alcoolique puisque la température lors de cette étape est de l'ordre de 30 à 35°C.

De plus, le fait de complémenter le génome de *Trichoderma reesei* directement par le lysozyme permet d'éviter d'introduire un agent bactéricide extérieur dans le procédé de production d'éthanol, cette souche étant déjà celle utilisée pour l'hydrolyse enzymatique.

De façon très préférée, le lysozyme utilisé pour complémenter le génome de *Trichoderma reesei* est le lysozyme humain ou de poulet.

Le lysozyme est une enzyme de type hydrolase (acide et sécrétée par les leucocytes) découverte par Alexander Fleming en 1922. Il s'agit d'une protéine globulaire de 129 acides aminés que l'on rencontre dans un certain nombre de sécrétions (larmes, salive...). Elle peut être aussi extraite du blanc d'oeuf ce qui caractérise son utilisation en oenologie. Utilisée depuis plusieurs années dans les industries pharmaceutiques et agroalimentaires, elle est rajoutée depuis peu dans certains processus d'oenologie (maturation du vin par exemple) afin de maîtriser la contamination par les bactéries lactiques. Cette enzyme agit en dégradant leur paroi (hydrolyse les liaisons covalentes entre l'acide N-acétyl muramique avec le quatrième atome de carbone du N-acétyl glucosamine). D'un point de vue biochimique, le lysozyme possède une activité optimale entre 40 et 45°C, ce qui est tout à fait compatible avec les températures utilisées pour l'hydrolyse enzymatique, et peut rester actif jusqu'à 62°C. En ce qui concerne le pH optimal, l'activité enzymatique de cette protéine n'est pas ou peu perturbée à l'intérieur d'une gamme de pH comprise entre 3,5 et 7, mais elle peut cependant rester active entre 2 et 10.

Le lysozyme est également employé comme conservateur dans beaucoup de produits alimentaires susceptibles de contenir des bactéries lactiques comme les fromages, le tofu ou le saké. L'utilisation du lysozyme dans les procédés brassicoles a recueilli tout récemment l'attention et les applications dans les étapes de brassage sont nombreuses. Le lysozyme est efficace contre toutes les bactéries lactiques. De plus, la levure et les bactéries Gram-négatives ne sont pas attaquées par cet agent antimicrobien.

Le lysozyme présente également l'avantage de limiter l'usage du SO₂, produit très souvent utilisé actuellement pour limiter les contaminations microbiologiques dans les procédés industriels mais coûteux. Le lysozyme peut également jouer un rôle protecteur en cas de fin de fermentation difficile. En effet, les carences en azote rendent les fins de fermentation alcoolique difficiles avec risque de développement de bactéries lactiques qui dégradent les oses non encore fermentés. L'ajout de lysozyme permet de prévenir ou traiter ce genre de problèmes, avec une grande efficacité.

Dans le cas du procédé de production d'éthanol par voie biologique selon la présente invention, l'avantage principal réside dans le fait que le lysozyme est une protéine qui peut être exprimée directement par *Trichoderma reesei.*

L'expression hétérologue de lysozyme humain a été développée dans le champignon filamenteux *Tolypocladium geodes* (Michel Baron "Optimisation au niveau moléculaire de souches transformées de Tolypocladium geodes pour la sécrétion de deux protéines humaines d'intérêt thérapeutique, (1991), Thèse de doctorat de l'université Paul Sabatier).

Dans l'optique d'une complémentation du génome de *T. reesei* par le gène *h*/*z* exprimant le lysozyme, une construction plasmidique modèle est réalisée en utilisant les méthodes classiques de biologie moléculaire connues de l'homme de l'art. La construction génétique à intégrer dans le génome de *T. reesei* comprend la séquence codante du lysozyme humain insérée entre les éléments permettant son expression et sa sécrétion chez le champignon. Dans le cadre d'une production conjointe aux cellulases, les séquences nucléotidiques doivent permettre l'expression hétérologue du gène *h*/*z.* Le promoteur, la séquence signal et le terminateur entourant ce gène doivent donc comprendre un ou plusieurs motifs connus par l'homme de l'art comme étant impliqués dans l'induction spécifique de ces enzymes. En particulier, le promoteur fongique utilisé peut être choisi, de façon non exclusive parmi les suivants : gpd (*A. nidulans), cbh1, eg*/*1, eg*/*2, xyn1.* La séquence signal fongique peut être choisie par exemple parmi *cbh1, eg*/*1, eg*/*2, xyn1.* Une fusion du lysozyme avec une protéine facilitant l'export (Sh-Ble par exemple), comme décrit dans la thèse de M. Baron, peut être utilisée. Le terminateur fongique peut être de toute nature, par exemple cbh1 ou TrpC (*A. nidulans*).

La construction doit enfin permettre d'identifier la souche ayant intégrée ce gène du lysozyme. Pour cela plusieurs gènes, conférant la résistance à un antibiotique (phléomycine, hygromycine etc.) ou permettant un criblage par auxotrophie (gène *amds* pour l'acétamide), sont connus par l'homme de l'art et peuvent être employés pour les souches industrielles de *T. reesei.*

Un exemple de construction plasmidique permettant la dite expression est décrit sur la Figure 1. Sur cette figure, les légendes utilisées sont les suivantes : "Prom" pour promoteur, "Term" pour terminateur, "bact" pour bactérie, "ss + HLZ" pour gène codant pour le lysozyme avec séquence d'adressage fonctionnel pour *T. reesei,* "Sh ble" pour gène conférant la résistance à l'antibiotique phléomycine.

La construction ainsi réalisée permet la sélection d'un transformant grâce à l'incorporation est ensuite incorporée au génome de la souche fongique déjà utilisée pour produire les cellulases, par exemple la souche hyperproductrice CL847 (Durand et al., 1984) selon les procédés déjà décrits et bien connus de l'homme de l'art, par exemple décrit par Penttila et al. (1987).

Les transformants obtenus sont sélectionnés pour une forte activité lysozyme, selon la méthode décrite précédemment par M. Baron (1991, Thèse de doctorat Université Paul Sabatier, Toulouse).

La souche sélectionnée sécrète en plus des enzymes cellulolytiques une proportion de lysozyme entre 1 et 5% des sécrétions protéiques de la souche, ce qui, dans le cadre d'une hydrolyse enzymatique type, par exemple réalisée à 20 mg/g de substrat et à 20% de matière sèche permet de se situer à une concentration de lysozyme dans la réaction d'hydrolyse située entre 40 et 200 mg/L, qui sont des proportions couramment employées dans l'industrie pour prévenir les contaminations de bactéries lactiques.

Les enzymes sont ensuite produites selon un procédé classique qui peut être de toute nature. Les enzymes sont séparées et utilisées pour la réaction d'hydrolyse comme décrit précédemment. Aucune autre précaution n'est à prendre pour éviter les contaminations bactériennes pour le reste du procédé, contrairement à ce qui doit être fait avec une souche classique.

L'action de l'agent bactéricide est d'autant plus efficace que la concentration en bactéries lactiques dans le milieu est faible. Dans le procédé selon la présente invention, l'agent bactéricide le lysozyme est produit par le microorganisme utilisé pour la sécrétion des enzymes employées lors de l'étape d'hydrolyse enzymatique. Or, à ce stade du procédé, en raison des conditions opératoires de température notamment, la prolifération lactique reste peu probable ou limitée, comparativement à celle qui peut avoir lieu lors de l'étape suivante de fermentation alcoolique.

Les matériaux cellulosiques ou lignocellulosiques utilisés dans le procédé selon la présente invention sont choisis parmi les pailles, le bois, les cultures forestières, les résidus de plantes alcooligènes, sucrières et céréalières, les résidus de l'industrie papetière et les produits de transformations des matériaux cellulosiques et lignocellulosiques.

Le matériau à hydrolyser est mis en suspension en phase aqueuse à raison de 6 à 25% de matière sèche, de préférence 10 à 20%, le pH est ajusté entre 4 et 5,5 (de préférence entre 4,5 et 5,2) et la température entre 40 et 60°C (de préférence entre 45 et 50°C). La réaction d'hydrolyse dure généralement de 15 à 48 heures selon l'efficacité du prétraitement réalisé, la composition du cocktail enzymatique et la quantité d'enzymes ajoutées. La réaction est suivie par dosage des sucres libérés. La solution sucrée obtenue est ensuite séparée de la fraction non hydrolysée par filtration ou centrifugation, puis est utilisée pour la fermentation éthanolique. L'étape de fermentation éthanolique se fait selon les connaissances générales de l'homme du métier, en présence de levures comme par exemple *Saccharomyces cerevisiae* ou *Zymomonas mobilis,* la température optimale de fermentation étant en général comprise entre 30-35°C.

Selon la présente invention, l'utilisation pour produire les enzymes cellulolytiques et/ou hémicellulolytiques d'une souche de *Trichoderma reesei* modifiée génétiquement et surexprimant peut être réalisée dans un procédé dans lequel l'étape d'hydrolyse enzymatique est distincte de l'étape de fermentation.

Selon un autre mode de réalisation, le procédé selon la présente invention est un procédé SSF (saccharification et fermentation simultanées) consistant à effectuer l'hydrolyse enzymatique et la fermentation alcoolique en une seule étape. Dans ce cas, la température de fonctionnement est relativement basse (environ 34°C) et par conséquent plus propice à la contamination bactérienne.

## Revendications

1. Procédé de production d'éthanol à partir de matériaux cellulosiques ou lignocellulosiques comprenant des étapes de prétraitement d'un substrat cellulosique ou lignocellulosique, d'hydrolyse enzymatique du substrat prétraité puis de fermentation alcoolique de l'hydrolysat obtenu, dans lequel on utilise pour produire les enzymes cellulolytiques et/ou hémicellulolytiques, une souche fongique modifiée génétiquement appartenant au genre *Trichoderma* et surexprimant le lysozyme.

2. Procédé selon la revendication 1 dans lequel la souche fongique appartient à l'espèce *Trichoderma reesei.*

3. Procédé selon l'une des revendications précédentes dans lequel les matériaux cellulosiques ou lignocellulosiques sont choisis parmi les pailles, le bois, les cultures forestières, les résidus de plantes alcooligènes, sucrières et céréalières, les résidus de l'industrie papetière et les produits de transformations des matériaux cellulosiques et lignocellulosiques.

4. Procédé selon l'une des revendications précédentes dans lequel l'étape d'hydrolyse enzymatique a lieu à une température comprise entre 40 et 60°C et un pH variant entre 4 et 5,5.

5. Procédé selon l'une des revendications précédentes dans lequel l'étape d'hydrolyse enzymatique est suivie d'une étape de fermentation éthanolique.

6. Procédé selon l'une des revendications 1 à 4 dans lequel l'étape d'hydrolyse enzymatique et la fermentation éthanolique sont réalisées simultanément.

## Claims

1. A method of producing ethanol from cellulosic or lignocellulosic materials, comprising stages of pretreatment of a cellulosic or lignocellulosic substrate, of enzymatic hydrolysis of the pretreated substrate and of alcoholic fermentation of the hydrolysate obtained, wherein a genetically engineered fungic strain belonging to the *Trichoderma* genus and overexpressing lysozyme is used to produce cellulolytic and/or hemicellulolytic enzymes.

2. A method as claimed in claim 1, wherein the fungic strain belongs to the *Trichoderma reesel* species.

3. A method as claimed in any one of the previous claims, wherein the cellulosic or lignocellulosic materials are selected from among straws, wood, forest crops, alcohol-producing crop, sugar crop and cereal crop residues, paper Industry residues, cellulosic and lignocellulosic material transformation products.

4. A method as claimed in any one of the previous claims, wherein the enzymatic hydrolysis stage is carried out at a temperature ranging between 40°C and 60°C and at a pH value ranging between 4 and 5.5.

5. A method as claimed in any one of the previous claims, wherein the enzymatic hydrolysis stage is followed by an ethanolic fermentation stage.

6. A method as claimed in any one of claims 1 to 5, wherein the enzymatic hydrolysis stage and the ethanolic fermentation stage are carried out simultaneously.

## Patentansprüche

1. Verfahren zur Herstellung von Ethanol aus zellulosehaltigen oder lignozellulosehaltigen Materialien, das Schritte zum Vorbehandeln eines zellulosehaltigen oder lignozellulosehaltigen Substrats, zum enzymatischen Hydrolysieren des vorbehandelten Substrats, dann zum alkoholischen Fermentieren des erhaltenen Hydrolysats umfasst, wobei zur Produktion der zellulosespaltenden und/oder hemizellulosespaltenden Enzyme ein gentechnisch modifizierter Pilzstamm verwendet wird, der zur Gattung *Trichoderma* gehört und das Lysozym überexprimiert.

2. Verfahren nach Anspruch 1, wobei der Pilzstamm zur Spezies *Trichoderma reesei* gehört.

3. Verfahren nach einem der vorhergehenden Ansprüche, wobei die zellulosehaltigen oder lignozellulosehaltigen Materialien ausgewählt sind aus Stroh, Holz, Forstkulturen, Rückständen von alkoholproduzierenden Pflanzen, Zuckerpflanzen und Getreidepflanzen, Rückständen der Papierindustrie und Transformationsprodukten der zellulosehaltigen und lignozellulosehaltigen Materialien.

4. Verfahren nach einem der vorhergehenden Ansprüche, wobei der Schritt zum enzymatischen Hydrolysieren bei einer Temperatur im Bereich zwischen 40 und 60 °C und bei einem pH-Wert, der zwischen 4 und 5,5 variiert, stattfindet.

5. Verfahren nach einem der vorhergehenden Ansprüche, wobei auf den Schritt zum enzymatischen Hydrolysieren ein Schritt zum ethanolischen Fermentieren folgt.

6. Verfahren nach einem der Ansprüche 1 bis 4, wobei der Schritt zum enzymatischen Hydrolysieren und zum ethanolischen Fermentieren gleichzeitig durchgeführt wird.
